(19) Europäisches Patentamt
European Patent Office
Office européen des brevets

(11) Veröffentlichungsnummer: **0 450 504 A1**

# (12) EUROPÄISCHE PATENTANMELDUNG

(21) Anmeldenummer: **91104953.4**

(22) Anmeldetag: **28.03.91**

(51) Int. Cl.5: **C07D 495/14**, C07D 495/04, B01D 15/08, //A61K31/55, (C07D495/14,333:00,235:00), (C07D495/04,333:00,243:00)

(30) Priorität: **02.04.90 DE 4010528**

(43) Veröffentlichungstag der Anmeldung:
**09.10.91 Patentblatt 91/41**

(84) Benannte Vertragsstaaten:
**AT BE CH DE DK ES FR GB GR IT LI LU NL SE**

(71) Anmelder: **BOEHRINGER INGELHEIM KG**

**W-6507 Ingelheim am Rhein(DE)**
(84) **BE CH DE DK ES FR GR IT LI LU NL SE AT**

Anmelder: **Boehringer Ingelheim G.m.b.H**

**W-6507 Ingelheim am Rhein(DE)**
(84) **GB**

(72) Erfinder: **Brandt, Klaus, Dr.**
**Georg-Scheuing-Strasse 22**
**W-6507 Ingelheim am Rhein(DE)**
Erfinder: **Nagel, Jürgen, Dr.**
**7 Cypress Lane Fox Hill Cond.**
**Ridgefield, Connecticut 06877(US)**

(54) **Präparativ chromatographisches Trennverfahren zur Herstellung enantiomerenreiner Hetrazepine.**

(57) Die Erfindung betrifft ein flüssigchromatographisches Trennverfahren zur Herstellung enantiomerenreiner Hetrazepine.



Rank Xerox (UK) Business Services

Die Erfindung betrifft ein präparativ chromatographisches Trennverfahren zur Herstellung entantiomerenreiner Hetrazepine.

Substituierte Hetrazepine der allgemeinen Formel

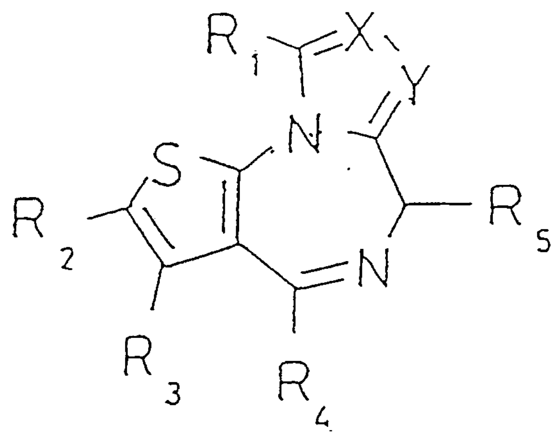

sind aus dem Stand der Technik als Arzneimittel mit PAF-antagonistischer Wirkung bekannt, so z.B. aus den europäischen Patentanmeldungen
EP-A-0.176,927, EP-A-0.176,928, EP-A-0.176.929,
EP-A-0.194.416, EP-A-0.230.942, EP-A-0.240.899,
EP-A-0.254.245, EP-A-0.255.028, EP-A-0.268.242,
EP-A-0.279.681, EP-A-0.284.359, EP-A-0.291.594,
EP-A-0.298.466, EP-A-0.315.698, EP-A-0.342.456,
EP-A-0.338 992, EP-A-0.328.924, EP-A-0.342.587,
EP-A-0.338 993, EP-O.368.175, EP-A-O.387.613,
EP-A-407.955,
auf die hiermit inhaltlich Bezug genommen wird.

Verbindungen des genannten Strukturtyps können - in Abhängigkeit der Definition der Substituenten $R_2$, $R_3$ oder $R_5$ ein optisch aktives Kohlenstoffatom enthalten. Im allgemeinen werden solche Verbindungen bei der Synthese - ausgehend von optisch inaktiven Ausgangsverbindungen - als Racemate erhalten. Es ist bekannt, daß die pharmakologische Wirkung der beiden Enantiomere nicht gleich ist, deshalb liegt es im Interesse einer modernen Arzneimittelforschung, lediglich das Enantiomere zu verwenden, das die stärkere pharmakologische Wirkung aufweist.

Es ist demnach die Aufgabe, ein im technischen Maßstab durchführbares Verfahren vorzuschlagen, das die Herstellung entantiomerenreiner Hetrazepine des oben genannten Strukturtyps ermöglicht.

Von besonderem Interesse sind hierbei enantiomerenreine Hetrazepine der allgemeinen Formel

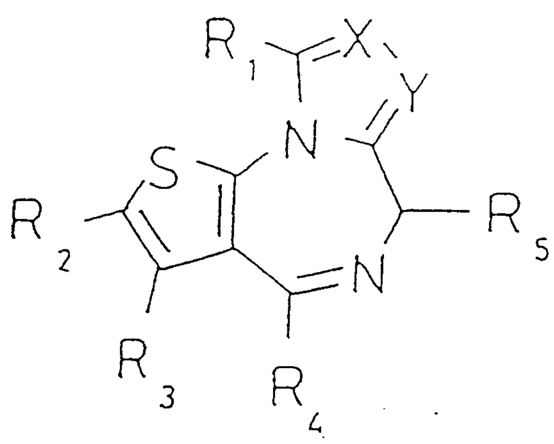

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, ein Cyclopropylgruppe, eine Cyclobutylgruppe, eine Cyclopentylgruppe, eine Cyclohexylgruppe, Halogen, bevorzugt Chlor und Brom;

$R_2$ einen Rest der Formel

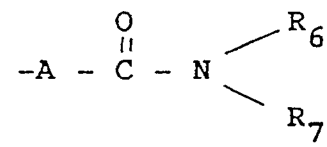

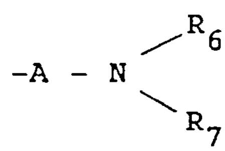

$-A - R_8$

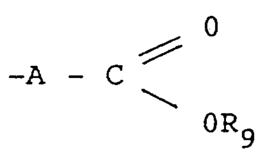

worin A eine verzweigte oder unverzweigte Alkylgruppe mit n Kohlenstoffatomen, wobei n eine der Zahlen 0, 1, 2, 3, 4, 5, 6, 7 oder 8

$R_6$ und $R_7$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit 1 bis 10 - bevorzugt 1 - 4 - Kohlenstoffatomen, die gegebenenfalls durch Halogen, Hydroxy, Nitro, Phenyl, substituiertes Phenyl, Amino, substituiertes Amino, $C_1$ bis $C_8$-bevorzugt $C_1$ bis $C_4$-Alkoxy substituiert sein kann;

$R_6$ oder $R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;

oder

$R_6$ und $R_7$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstofatomen substituierten 5-, 6- oder 7-Ring, der als weitere Heteroatome Stickstoff, Sauerstoff oder Schwefel enthalten kann, wobei jede weitere Stickstoffatom durch eine verzweigte oder unverzweigte Alkylgruppe mit 1 bis 4 Kohlenstoffatomen, bevorzugt Methyl, substituiert sein kann;

$R_8$ Phenyl, substituiertes Phenyl;

$R_9$ Wasserstoff, $C_1$ bis $C_4$ Alkyl;

$R_3$ Wasserstoff, $C_1$ - $C_4$ - Alkyl;

$R_4$ Phenyl, wobei der Phenylring ein- oder mehrfach, bevorzugt Halogen, Nitro und/oder Trifluormethyl substituiert sein kann;

$R_5$ Hydroxy, $C_1$ - $C_4$ - Alkyl, gegebenenfalls durch Hydroxy oder Halogen substituiert

oder

$R_2$ und $R_3$ bilden zusammen einen ankondensierten fünf- oder sechsgliedrigen Ring der Formel

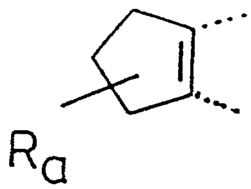
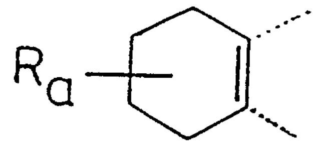

worin Ra einen Rest der Formel

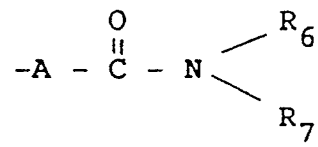

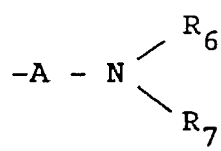

$-A - R_8$

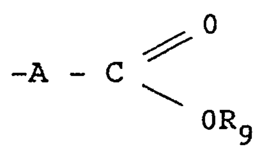

worin A, $R_6$, $R_7$, $R_8$ und $R_9$ die zuvor genannte Bedeutung aufweisen und $R_5$ Wasserstoff, Hydroxy, $C_1$-$C_4$-Alkyl, gebenenfalls durch Hydroxy oder Halogen substituiert bedeutet und X und Y beide Stickstoff oder X, CH und Y Stickstoff bedeuten können.

Bevorzugt sind solche Verbindungen der allgemeinen Formel 1, worin

$R_1$ = Methyl,

$R_2$ = $-CH_2-CH_2-CONR_6R_7$,

$R_2$ = $-CH_2-CH_2-NR_6R_7$,

$R_2 = -CH_2-CH_2-$ 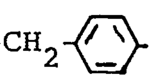 -iso-Butyl ,

$R_3$ = Wasserstoff, $R_5$ = Methyl,

$R_2$ und $R_3$ zusammen

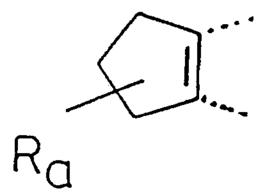
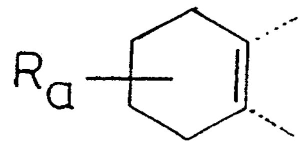

mit

Ra = $CONR_6R_7$,
$R_5$ = Wasserstoff, oder Methyl,
$R_4$ = ortho Chlorphenyl,
besonders bevorzugt ist $R_6/R_7 = C_3H_7$
oder zusammen mit dem Stickstoffatom einen Morpholinorest bedeuten.

Verbindungen der allgemeinen Formel I worin $R_2$ und $R_3$ einen ankondensierten 5- oder 6-Ring bilden und $R_5$ ungleich Wasserstoff ist enthalten zwei optisch aktive C-Atome im Ringsystem und bilden Diastereomerengemische. Diese können nach üblichen Verfahren, z. B. durch säulenchromatographische Trennung an $SiO_2$ als stationäre Phase und Essigester /Methanol als mobile Phase, in ihre Diastereomeren getrennt werden. Das erfindungsgemäße Verfahren ermöglicht dann die Trennung der Diastereomere in ihre Enantiomere.

Als Alkylgruppen (auch soweit sie Bestandteil anderer Reste sind) werden beispielsweise genannte: Methyl, Ethyl, Propyl, iso-Propyl, Butyl, iso-Butyl, sec. Butyl, tert.-Butyl, Pentyl, iso-Pentyl, Hexyl, Heptyl, Octyl, Nonyl und Dekanyl.

Als Alkenylgruppen werden beispielsweise oben genannte Alkylgruppen bezeichnet soweit sie mindestens eine Doppelbindung aufweisen, wie zum Beispiel Vinyl (soweit keine unbeständigen Enamine gebildet werden), Propenyl, iso-Propenyl, Butenyl, Pentenyl, Hexenyl.
Als Alkinylgruppen werden beispielsweise oben genannte Alkylgruppen bezeichnet, soweit sie mindestens eine Dreifachbindung aufweisen, wie zum Beispiel Propargyl, Butinyl, Pentinyl, Hexinyl.
Als Cycloalkylreste mit 3 - 6 Kohlenstoffatomen werden beispielsweise Cyclopropyl, Cyclobutyl, Cyclopentyl oder Cyclohexyl bezeichnet, die durch verzweigtes oder unverzweigtes Alkyl mit 1 bis 4 Kohlenstoffatomen, Hydroxy, und/oder Halogen substituiert sein können.

Beispielhaft für substituiertes Phenyl werden genannt:
3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert.Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimthoxyphenyl, 3,4,5-Trimethoxyphenyl, 2-Chlorbenzyl, 2,3-Dichlorbenzyl, 2-Methylbenzyl, 2-Trifluormethylbenzyl, 4-Methoxybenzyl, 3,4,5-Trimethoxybenzyl, 2-(2-Chlorphenyl)ethyl,
Beispiele für gegebenenfalls substituierte gesättigte oder ungesättigte heterocyclische 5-, 6- oder 7-gliedrige Ringe bzw. Heteroaryleste sind:
Pyrrol, Pyrrolin, Pyrrolidin, 2-Methylpyrrolidin, 3-Methylpyrrolidin, Piperidin - gegebenenfalls durch $C_1$ - $C_4$ Alkyl ein- oder mehrfach substituiert - Piperazin, N-Methylpiperazin, N-Ethylpiperazin, N-N-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;
Als heterocyclische Reste, die über ein Kohlenstoffatom verknüpft sein können, werden beispielsweise Thiophen, 2-Methylthiophen, Furan, Tetrahydrofuran, 2-Methyltetrahydrofuran, Tetrahydrofuan, 2-Hydroxymethylfuran, α-Pyran, γ-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy oder Halogen substituiert sein kann.

Als Heterocyclus im Rahmen der zuvor angegebenen Definition steht im allgemeinen auch für einen 5- bis 6-gliedrigen Ring, der als Heteroatome Sauerstoff, Schwefel und/oder Stickstoff enthalten kann, wie zum Beispiel Thienyl, Furyl, Pyridyl, Pyrimidyl, Pyrazinyl, Pyrazinyl, Chinolyl, Isochinolyl, Chinazolyl, Chinoxalyl, Thiozolyl, Benzothiazolyl, Isothiazolyl, Oxazolyl, Benzooxazolyl, Isoxazolyl, Imidazolyl, Benzimidazolyl, Pyrazolyl und Indolyl genannt.

Erfindungsgemäß können Racemate der allgemeinen Formel I durch ein flüssigchromatographisches Verfahren in ihre Enantiomeren getrennt werden, bei dem Cellulose-triacetat als stationäre Phase eingesetzt

wird. Ein geeignetes Cellulosetriacetat ist beispielsweise von der Firma Merck in D-6100 Darmstadt unter der Bestellnummer 16 363.0250 erhältlich.

Als mobile Phase verwendet man polare Lösungsmittel oder Lösungsmittelgemische, wie z.B. Methanol, Ethanol, Methanol-Wasser, Ethanol-Wasser, Ethanol-Hexan, Ethanol-Isopropanol-Hexan, bevorzugtes Lösungsmittel ist Methanol.

Bevorzugtes Chromatographieverfahren ist die Hochdruck - Flüssigkeits - Chromatographie (HPLC), geeignet - wenn auch Zeitaufwendiger - sind jedoch auch Niederdruck und Mitteldruckchromatographie. Im Gegensatz zum Stand der Technik, EP 254 245), der lediglich eine Trennung in analytischen Mengen beschreibt, ermöglicht das erfindungsgemäße Verfahren eine Trennung im technischen Maßstab.

Die nachfolgenden Beispiele sollen die Erfindung erläutern, ohne sie jedoch in ihrem Umfang zu beschränken.

Beispiel 1:

80 g (R,S) 6-(2-Chlorphenyl)-8,9-dihydro-1-methyl-8-[(4-morpholinyl)carbonyl]-4H,7H-cyclopenta[4,5)-thieno-[3,2-f][1,2,4]triazolo[4,3-a)[1,4]diazepin (WEB 2170) werden in 80 ml Methanol im Ultraschallbad gelöst. Die Lösung wird auf eine Cellulosetriacetatsäule Teilchendurchmesser 25 - 40 µm der Größe 500 mm x 200 mm i.D. aufgegeben. Mit einem Fluß von 150 ml/min reinem Methanol wird das WEB 2170 über die Säule gefördert und dabei in die Enantiomeren getrennt. Zur Detektion verwendet man einen Durchflußpolarimeterdetektor. Aus dem vorlaufenden Eluat erhält man nach üblicher Aufarbeitung 25 g (% d. Th.) des (-)-Isomeren vom Drehwert $\alpha_D^{20}$ = - 12,00.

Aus dem zweiten Eluat werden 20 g (% d. Th.) des (+)-Isomeren vom Drehwert $\alpha_D^{20}$ = + 9,50 isoliert.

In Analogie zu Beispiel 1 werden die folgenden Verbindungen in ihre Enantiomeren getrennt.

Beispiel 2:

(R,S)-6-(2-Chlorphenyl)-8,9-dihydro-8-(N,N-dipropylaminocarbonyl)-1-methyl-4H,7H-cyclopenta[4,5]-thieno[3,2-f]-[1,2,4]triazolo[4,3-a][1,4]diazepin

Beispiel 3:

(R,S)-6-(2-Chlorphenyl)-1-cyclopentyl-8,9-dihydro-8-(morpholinocarbonyl)-4H,7H-cyclopenta[4,5]thieno-[3,2-f] [1,2,4]triazolo[4,3-a][1,4]diazepin

Beispiel 4:

(R,S)-6-(2-Chlorphenyl)-8,9-dihydro-1-methyl-8-(morpholinocarbonyl)-4H,7H-cyclohexa[4,5]thieno.[3,2-f] [1,2,4]triazolo[4,3-a][1,4]diazepin

Beispiel 5:

(R,S)-6-(2-Chlorphenyl)-8,9-dihydro-1-methyl-8-(morpholinocarbonyl)-4H,7H,cyclopenta[4,5]thieno-[3,2-f] [1,4]imidazolo[4,3-a](1,4]diazepin

Die nachfolgenden Abbildungen zeigen die Chromatogramme oben genannter Verbindungen.

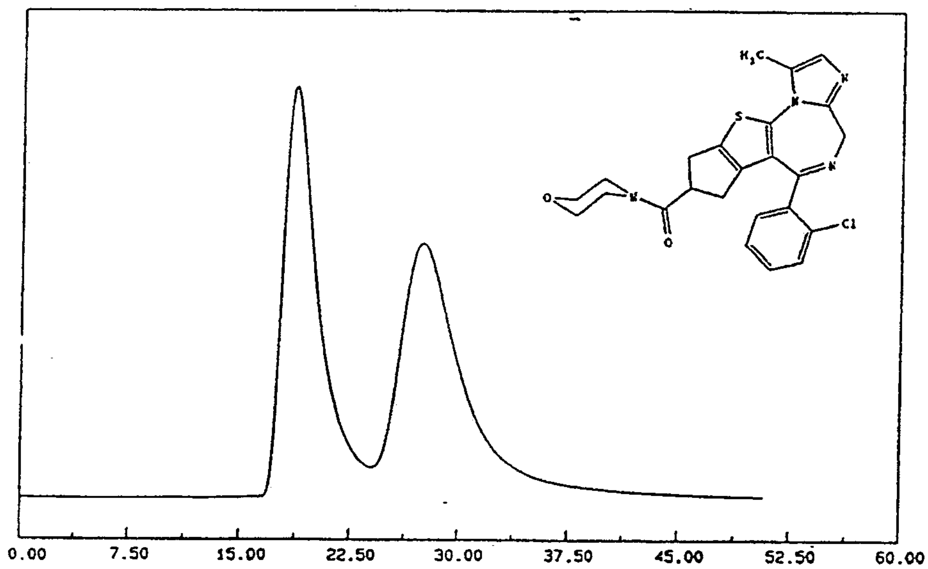
0.00
7.50
15.00
22.50
30.00
37.50
45.00
52.50
60.00

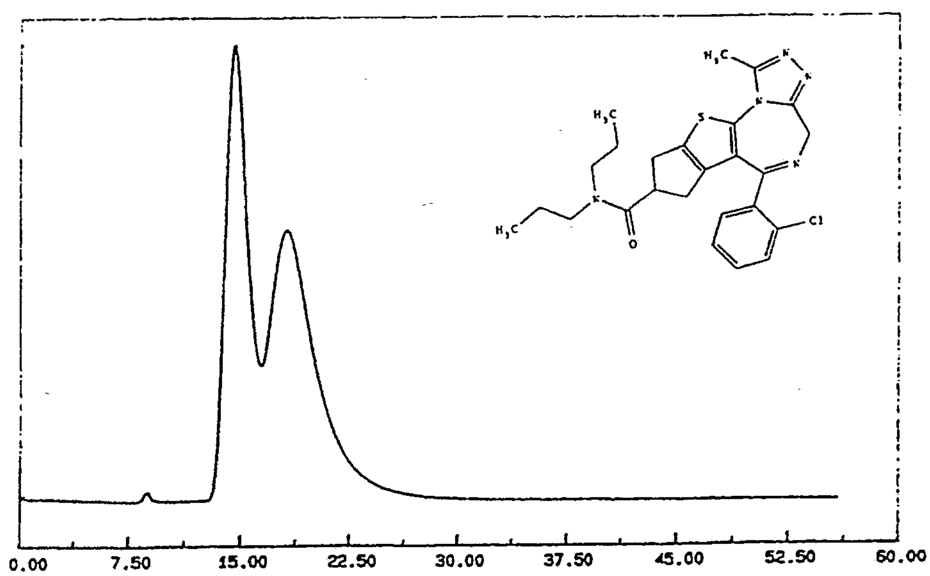
0.00
7.50
15.00
22.50
30.00
37.50
45.00
52.50
60.00

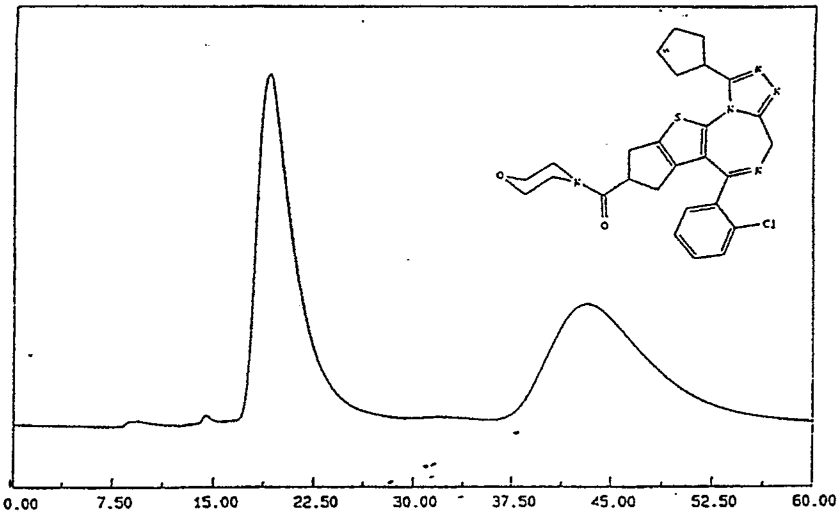


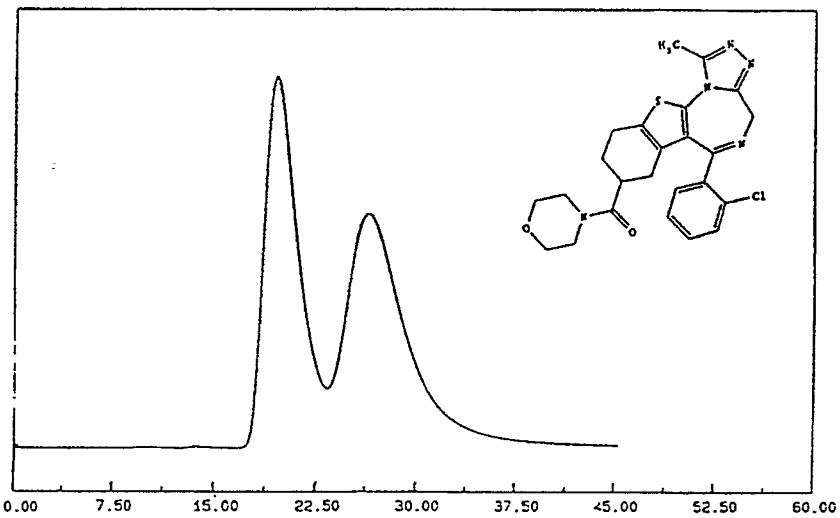


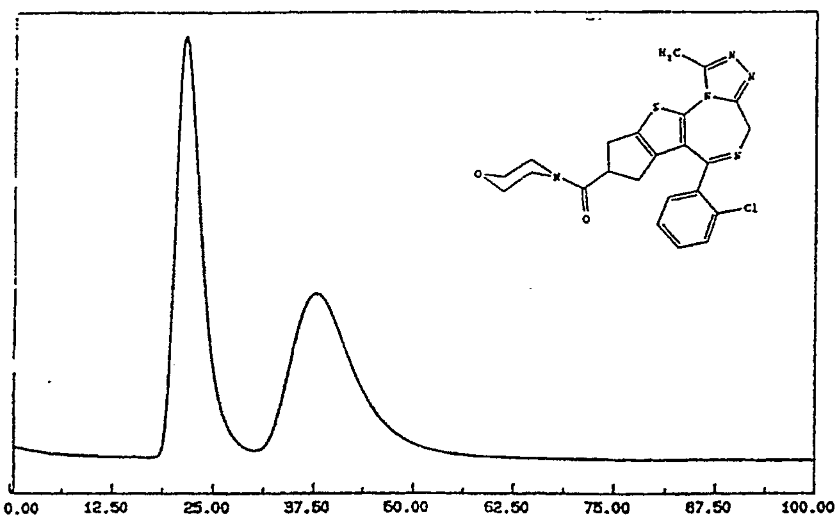


## Patentansprüche

1. Flüssigchromatographisches Trennverfahren zur Herstellung enantiomerenreiner Hetrazepine, die ein Strukturelement der allgemeinen Formel

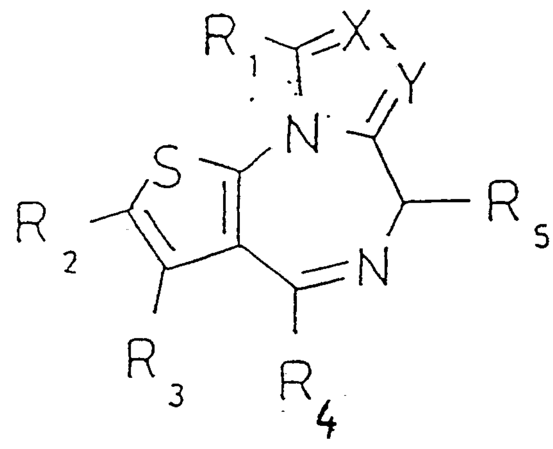

enthalten, worin X und Y unabhängig voneinander Stickstoff, C-H oder $CH_3$ und $R_1$ bis $R_5$ Substituenten, $R_2$ und $R_3$ auch ankondensierte, gegebenenfalls substituierte Ringsysteme bedeuten können, ausgehend von den entsprechenden racemischen Hetrazepinen der allgemeinen Formel I, dadurch gekennzeichnet, daß als stationäre Phase Cellulosesetriacetat und als flüssige Phase ein polares Lösungsmittel- oder Lösungsmittelgemisch verwendet wird.

**2.** Flüssigchromatographisches Trennverfahren zur Herstellung enantiomerenreiner Hetrazepine der allgemeinen Formel

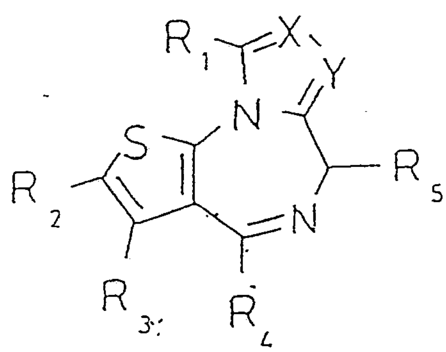

I

worin $R_1$, $R_2$, $R_3$, $R_4$, $R_5$, X und Y wie in einer der nachfolgenden Europäischen Patentanmeldungen

EP-A-0.176,927, EP-A-0.176,928, EP-A-0.176.929,
EP-A-0.194.416, EP-A-0.230.942, EP-A-0.240.899,
EP-A-0.254.245, EP-A-0.255.028, EP-A-0.268.242,
EP-A-0.279.681, EP-A-0.284.359, EP-A-0.291.594,
EP-A-0.298.466, EP-A-0.315.698, EP-A-0.342.456,
EP-A-0.338.992, EP-A-0.328.924, EP-A-0.342.587,
EP-A-0.338.993, EP-A-O.368.175, EP-A-O.387.613,
EP-A-O.407.955,

definiert sind,
dadurch gekennzeichnet, daß das als stationäre Phase Cellusosetriacetat verwendet wird und als flüssige Phase ein polares Lösungsmittel- oder Lösungsmittelgemisch verwendet wird.

**3.** Verfahren gemäß Anspruch 1 oder 2 zur Enantiomerentrennung von Verbindungen der allgemeinen Formel I

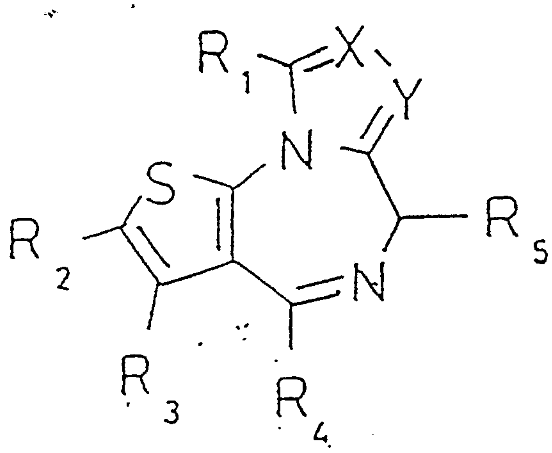

worin

$R_1$ Wasserstoff, eine verzweigte oder unverzweigte Alkylgruppe mit 1 bis 4 Kohlenstoffatomen, bevorzugt Methyl, die gegebenenfalls durch Hydroxy oder Halogen substituiert sein kann, ein Cyclopropylgruppe, eine Cyclobutylgruppe, eine Cyclopentylgruppe, eine Cyclohexylgruppe, Halogen, bevorzugt Chlor und Brom;

$R_2$ einen Rest der Formel

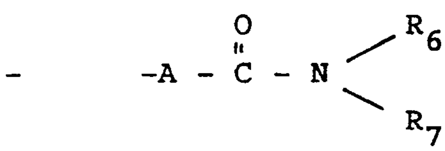

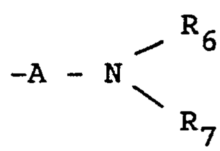

$$-A - R_8$$

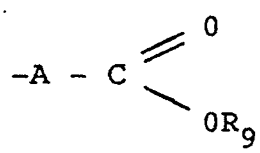

worin A eine verzweigte oder unverzweigte Alkylgruppe mit n Kohlenstoffatomen, wobei n eine der Zahlen 0, 1, 2, 3, 4, 5, 6, 7 oder 8

$R_6$ und $R_7$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl- oder Alkinylgruppe mit 1 bis 10 - bevorzugt 1 - 4 - Kohlenstoffatomen, die gegebenenfalls durch Halogen, Hydroxy, Nitro, Phenyl, substituiertes Phenyl, Amino, substituiertes Amino, $C_1$ bis $C_8$- bevorzugt $C_1$ bis $C_4$-Alkoxy substituiert sein kann;

$R_6$ oder $R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;

oder

$R_6$ und $R_7$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstofatomen substituierten 5-, 6- oder 7-Ring, der als weitere Heteroatome Stickstoff, Sauerstoff oder Schwefel enthalten kann, wobei jede weitere Stickstoffatom durch eine verzweigte oder unverzweigte Alkylgruppe mit 1 bis 4 Kohlenstoffatomen, bevorzugt Methyl, substituiert sein kann;

- $R_8$ Phenyl, substituiertes Phenyl;
- $R_9$ Wasserstoff, $C_1$ bis $C_4$ Alkyl;
- $R_3$ Wasserstoff, $C_1$ - $C_4$ - Alkyl
- $R_4$ Phenyl, wobei der Phenylring ein- oder mehrfach, bevorzugt Halogen, Nitro und/oder Trifluormethyl substituiert sein kann;
- $R_5$ Hydroxy, $C_1$ - $C_4$ - Alkyl, gegebenenfalls durch Hydroxy oder Halogen substituiert

oder

$R_2$ und $R_3$ bilden zusammen einen ankondensierten fünf- oder sechsgliedrigen Ring der Formel

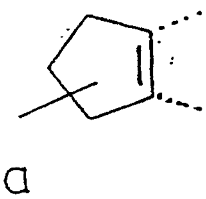

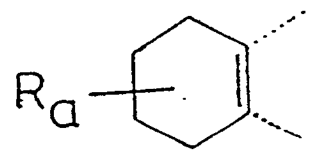

worin Ra einen Rest der Formel

– 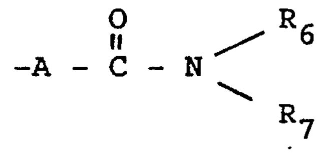

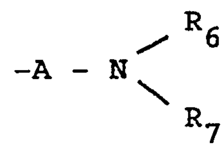

$-A - R_8$

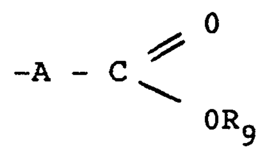

worin A, $R_6$, $R_7$, $R_8$ und $R_9$ die zuvor genannte Bedeutung aufweisen und

$R_5$ Wasserstoff, Hydroxy, $C_1$-$C_4$-Alkyl, gebenenfalls durch Hydroxy oder Halogen substituiert bedeutet, und X und Y beide Stickstoff oder X = CH und Y = Stickstoff bedeutet.

**4.** Verfahren zur Trennung von (R,S) 6-(2-Chlorphenyl)-8,9-dihydro-1-methyl-8-[(4-morpholinyl)carbonyl]-4H,7H-cyclopenta[4,5]thieno[3,2-f][1,2,4]triazolo-[4,3-a][1,4]diazepin (WEB 2170) dadurch gekennzeichnet, daß als stationäre Phase Cellulosetriacetat und als flüssige Phase ein polares Lösungsmittel oder Lösungsmittelgemisch verwendet wird.

**5.** Verfahren nach Anspruch 1, 2, 3 oder 4, dadurch gekennzeichnet, daß als Lösungsmittel Methanol, Ethanol, Methanol-Wasser, Ethanol-Wasser, Ethanol-Hexan oder Ethanol-Isopropanol-Hexan verwendet wird.

**6.** Verwendung von Cellulosetriacetat als Säulenfüllmaterial zur flüssigkeitschromatographischen Trennung racemischer Hetrazepine der allgemeinen Formel I wie in Anspruch 1, 2, 3 oder 4 definiert.

Europäisches Patentamt

# EUROPÄISCHER RECHERCHENBERICHT

Nummer der Anmeldung

EP 91104953.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
| D,A | EP - A1 - 0 254 245 (BOEHRINGER INGELHEIM KG) * Ansprüche 1,4; Seite 18, Zeilen 17-19 * -- | 1-3 | C 07 D 495/14<br>C 07 D 495/04<br>B 01 D 15/08<br>//A 61 K 31/55<br>(C 07 D 495/14<br>C 07 D 333:00<br>C 07 D 235/00)<br>(C 07 D 495/04<br>C 07 D 333:00<br>C 07 D 243:00) |
| D,A | EP - A2/A3 - 0 230 942 (BOEHRINGER INGELHEIM KG) * Ansprüche 1,5; Seite 16, Zeilen 47-48 * ---- | 1-3 | |
| | | | RECHERCHIERTE SACHGEBIETE (Int. Cl.⁵) |
| | | | C 07 D 495/00 |

Der vorliegende Recherchenbericht wurde für alle Patentansprüche erstellt.

| Recherchenort | Abschlußdatum der Recherche | Prüfer |
|---|---|---|
| WIEN | 13-05-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62